# NEUE EUROPÄISCHE PATENTSCHRIFT

(11) **EP 0 966 922 B2**
(45) Veröffentlichungstag und Bekanntmachung des Hinweises auf die Entscheidung über den Einspruch: **25.08.2010**
(45) Hinweis auf die Patenterteilung: 16.04.2003
(21) Anmeldenummer: 99110269.0
(22) Anmeldetag: 27.05.1999
(51) Int. Cl.: A61B 17/34, A61N 1/36, A61N 1/34

(54) **Unipolar-Kanüle für die kontinuierliche Leitungsanästhesie**
Unipolar cannula for continuous anaesthesia
Canule unipolaire pour anesthésie continue

(30) Priorität: 27.06.1998 DE 19828794
(43) Veröffentlichungstag der Anmeldung: 29.12.1999
(73) Patentinhaber: Pajunk, Heinrich, 78187 Geisingen (DE); Pajunk, Horst, 78187 Geisingen (DE)
(72) Erfinder: Pajunk, Heinrich, 78187 Geisingen (DE); Pajunk, Horst, 78187 Geisingen (DE); Meier, Gisela Dr.med., 82418 Murnau (DE)
(74) Vertreter: Patentanwälte Westphal, Mussgnug & Partner

(56) Entgegenhaltungen:
- EP-A- 0 102 538
- WO-A-99/36113
- DE-A- 4 420 232
- DE-C- 3 643 235
- DE-U- 8 803 153
- US-A- 5 306 236

## Beschreibung

Die Erfindung betrifft eine Unipolar-Kanüle für die kontinuierliche Leitungsanästhesie.

Es ist eine Unipolar-Kanüle für die kontinuierliche Leitungsanästhesie der Firma Pajunk GmbH, D-78187 Geisingen, bekannt mit einem elektrisch leitenden Kanülenrohr, welches eine elektrisch isolierende äußere Beschichtung aufweist, die sich von einem proximalen Ansatz bis zu der distalen Spitze des Kanülenrohres erstreckt und die distale Spitze in ihrem distalen Endbereich freiläßt. Im Bereich des proximalen Ansatzes ist das Kanülenrohr durch einen Anschluß elektrisch kontaktiert, welcher über eine Buchse an ein Elektrostimulationsgerät angeschlossen werden kann. Die distale Spitze des Kanülenrohres ist entweder mit einem Facettenschliff versehen oder ist als sogenannte Sprotte-Spitze gemäß DE 30 20 926 C2 ausgebildet.

Bei dieser bekannten Unipolar-Kanüle sind der Anschluß für die Elektrostimulation und ein Zuspritzschlauch für ein Anästhetikum achsparallel nebeneinander in die proximale Stirnfläche des Ansatzes des Kanülenrohres eingeführt. Die Unipolar-Kanüle kann mit ihrer distalen Spitze mittels der Elektrostimulation exakt in der Nervenscheide plaziert werden, um über den Zuspritzschlauch das Anästhetikum gezielt an den Nerv zu applizieren.

Bei der kontinuierlichen Leitungsanästhesie wird ein Katheter in der Nervenscheide plaziert, um das Anästhetikum über eine längere Zeitdauer zuführen zu können. Um den Katheter zu legen, ist es aus der DE-U-8803153 bekannt, auf das Kanülenrohr eine Kunststoff-Verweilkanüle zu ziehen, die mittels des elektrisch leitenden Kanülenrohres unter Elektrostimulation plaziert wird. Sobald die Kunststoff-Verweilkanüle gelegt ist, wird das Kanülenrohr herausgezogen und über die Kunststoff-Verweilkanüle kann der Katheter eingeführt werden. Der Oberbegriff des ersten Anspruchs basiert auf diesem Dokument.

Weiter ist es aus der DE -A- 46 43 235 bekannt, eine Kanüle mit Sprotte-Spitze so auszubilden, daß durch diese Kanüle selbst ein Katheter gelegt werden kann. Hierzu ist im Inneren der distalen Spitze des Kanülenrohres eine Rampe ausgebildet, die zu der seitlichen Austrittsöffnung führt. Ein proximal in das Kanülrohr eingeführter Katheter wird über diese Rampe durch die seitliche Austrittsöffnung aus dem Kanülenrohr herausgeführt. Diese Kanüle eignet sich zwar zum Plazieren eines Katheters ohne zusätzliche Verweilkanüle. Eine Elektrostimulation ist mit dieser bekannten Kanüle jedoch nicht möglich. Dementsprechend weist diese Kanüle keinen elektrischen Anschluß für die Elektrostimulation auf.

Aus der US-A-5306236 ist eine Spritzenkanüle bekannt, die eine isolierende Beschichtung aufweist und an deren proximalem Ende ein elektrischer Anschluß angebracht ist, so daß die Spitze der Spritzenkanüle durch Elektrostimulation positioniert werden kann.

Die Erfindung liegt die Aufgabe zugrunde, eine Unipolar-Kanüle für die kontinuierliche Leitungsanästhesie zur Verfügung zu stellen, die bei einfachem Aufbau und einfacher Handhabung das Plazieren eines Katheters mit den Vorteilen der Elektrostimulation vereinigt.

Diese Aufgabe wird erfindungsgemäß gelöst durch eine Unipolar-Kanüle mit den Merkmalen des Anspruchs 1.

Vorteilhafte Ausführungen und Weiterbildungen der Erfindung sind in den Unteransprüchen angegeben.

Die erfindungsgemäße Unipolar-Kanüle kann mit Hilfe der elektrischen Nervenstimulation plaziert werden. Die äußere isolierende Beschichtung des Kanülenrohres, die nur einen sehr kleinen, nahezu punktförmigen Bereich an der Spitze freiläßt erlaubt dabei eine äußerst präzise Plazierung der Spitze. Die Unipolar-Kanüle kann selbst zum Einführen des Katheters verwendet werden, wozu der am proximalen Ende des Kanülenrohres angeordnete Ansatz eine Einführöffnung aufweist, die axial fluchtend in das Kanülenrohr führt. Der Anschluß für die Elektrostimulation ist seitlich in den Ansatz eingeleitet und kontaktiert das elektrisch leitende Kanülenrohr von außen. Der Anschluß behindert und beeinträchtigt somit nicht die axiale Einführöffnung des Ansatzes. Nach dem Plazieren der Unipolar-Kanüle mit Hilfe der Elektrostimulation kann somit der Katheter durch das Kanülenrohr eingeführt werden, ohne daß hierzu die Lage der Unipolar-Kanüle geändert oder sonstige Maßnahmen vorgenommen werden müssen. An dem Ansatz mit der Einführöffnung ist ein lösbarer Anschluß ausgebildet, nämlich ein Luer-Lock-Anschluß. An diesen Anschluß kann nach Bedarf ein Zuspritzschlauch konnektiert werden, um ein Anästhetikum für eine erste oder eine kurzzeitige Anästhesie einzuleiten. Ebenso kann an den lösbaren Anschluß eine Spritze konnektiert werden, um ein Anästhetikum zu injizieren oder auch um Flüssigkeit zur Lagekontrolle zu aspirieren. Die Möglichkeit, den Ansatz sowohl zum alternativen Konnektieren eines Zuspritzschlauches oder eine Spritze als auch zum Einführen des Katheters zu verwenden, macht die Unipolar-Kanüle äußerst vielseitig. Diese Vielseitigkeit wird dabei mit einer äußerst einfachen und kostengünstigen Gestaltung erreicht. Die Handhabung der Unipolar-Kanüle ist ebenfalls äußerst einfach, da diese ohne Lageveränderung sowohl für die Injektion oder Aspiration als auch für das Einführen des Katheters genutzt werden kann. Das axial fluchtende Konnektieren einer Spritze an dem proximalen Ansatz ermöglicht auch eine Durchführung der Nervenblockade in der Einhandtechnik.

Die Spitze des Kanülenrohres kann mit einem Facettenschliff ausgebildet sein, so daß die Austrittsöffnung durch die schräg gegen die Kanülenachse geneigte Anschlifffläche gebildet wird.

In dieser Ausführung tritt der Katheter axial fluchtend aus der distalen Spitze des Kanülenrohres aus. Diese Ausführung eignet sich beispielsweise für die kontinuierliche anteriore Ischiadicusblockade, für das Legen eines distalen Ischiadicuskatheters oder für das Setzen eines Psoaskompartmentblokkes.

Ebenso kann die distale Spitze des Kanülenrohres als Sprotte-Spitze ausgebildet sein, wobei der durch das Kanülenrohr eingeführte Katheter mittels einer Rampe durch die seitlich hinter der Spitze angeordnete Austrittsöffnung geleitet wird. Der Katheter tritt hierbei unter einem Winkel von ca. 30° zur Kanülenachse aus. Dies ist bei den Anästhesie-Techniken von Vorteil, bei welchen eine Stichrichtung im wesentlichen parallel zu dem Nerv nicht möglich ist. Diese Ausführung der Unipolar-Kanüle wird beispielsweise angewendet bei der interscalenären Plexusblockade, der vertikal-infraclaviculären Plexusblockade, der Ischiadicusblockade und der Blockade des Nervus suprascapularis.

Im folgenden wird die Erfindung anhand von in der Zeichnung dargestellten Ausführungsbeispielen näher erläutert. Es zeigen
- Figur 1: eine Ansicht der Unipolar-Kanüle in einer ersten Ausführung,
- Figur 2: einen vergrößerten Axialschnitt dieser Unipolar-Kanüle, und
- Figur 3: einen Figur 2 entsprechenden Axialschnitt einer zweiten Ausführung der Unipolar-Kanüle.

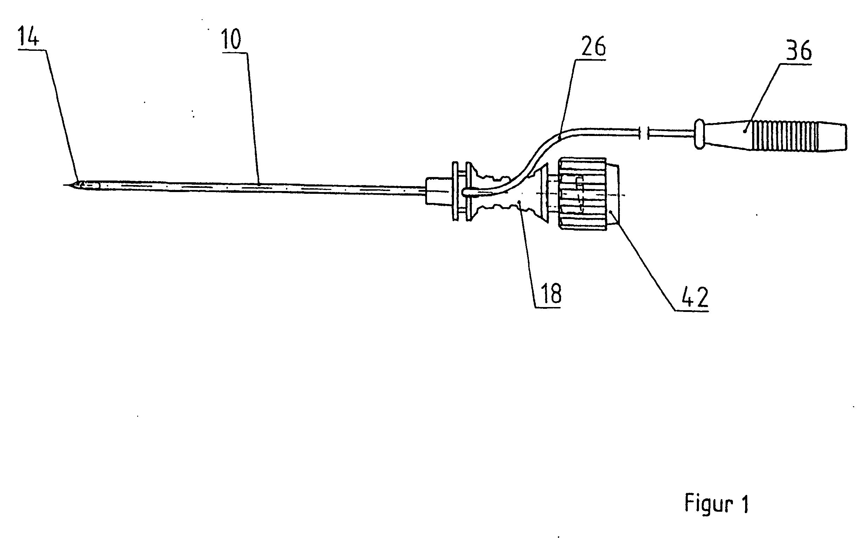

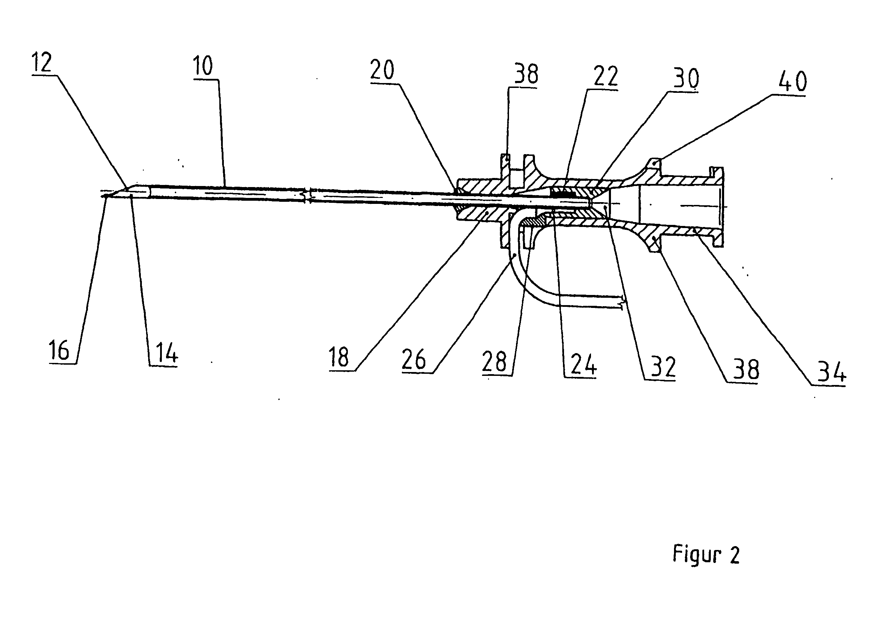

Die Figuren 1 und 2 zeigen ein erstes Ausführungsbeispiel der Unipolar-Kanüle.

Die Kanüle weist ein elektrisch leitendes Kanülenrohr 10 auf, welches vorzugsweise aus Stahl gefertigt ist. Je nach Modell weist das Kanülenrohr 10 eine Länge von 25 bis 200mm und einen Durchmesser von 0,5 bis 1,0 mm auf. In dem Ausführungsbeispiel der Figuren 1 und 2 ist das distale Ende des Kanülenrohres 10 mit einem Facettenschliff 12 unter 45° zur Achse des Kanülenrohres 10 angeschliffen, so daß eine distale Spitze 14 gebildet ist. Die äußere Mantelfläche des Kanülenrohres 10 ist mit einem elektrisch isolierenden Kunststoff beschichtet. Die Beschichtung erstreckt sich vom proximalen Ende des Kanülenrohres 10 bis zu dessen distaler Spitze 14 und läßt nur einen distalen Endbereich 16 der Spitze 14 mit einer Länge von maximal etwa 1mm frei, in welchem das Metall des Kanülenrohres 10 blank liegt.

Das proximale Ende des Kanülenrohres 10 ist koaxial in einen Ansatz 18 aus Kunststoff eingepaßt und mit diesem mittels eines aushärtenden Klebstoffes 20 verklebt. Der Ansatz 18 weist eine im wesentlichen zylindrische Form auf und ist koaxial von einer Bohrung durchsetzt. Im distalen Bereich entspricht der Innendurchmesser dieser Bohrung dem Außendurchmesser des in diese Bohrung eingepaßten Kanülenrohres 10. Das Kanülenrohr 10 ragt mit seinem proximalen Ende axial bis etwa in die Mitte des Ansatzes 18. Im Bereich des proximalen Endes des Kanülenrohres 10 erweitert sich der Innendurchmesser des Ansatzes 18, so daß zwischen der Innenwandung des Ansatzes 18 und dem Kanülenrohr 10 ein Ringspalt freibleibt. Im Bereich dieses Ringspaltes ist auf das metallische Kanülenrohr 10 in elektrisch leitendem Kontakt mit diesem eine zylindrische Anschlußbuchse 22 aufgepreßt. Mit dieser metallischen Anschlußbuchse 22 ist die leitende Ader 24 einer Anschlußlitze 26 verlötet. Die abisolierte Ader 24 verläuft in dem Bereich, in welchem sie mit der Anschlußbuchse 22 verlötet ist, achsparallel zu dem Kanülenrohr 10 in distaler Richtung. Die isolierte Anschlußlitze 26 biegt dann aus dieser achsparallelen Richtung rechtwinklig ab und tritt radial durch den Ansatz 18 nach außen. Die Öffnung des Ansatzes 18, durch welche die Anschlußlitze 26 austritt ist mittels eines aushärtenden Klebstoffes 28 vergossen.

Der Ringspalt zwischen der Innenwandung der Bohrung des Ansatzes 18 und dem proximalen Ende des Kanülenrohres 10 mit der Anschlußbuchse 22 und der Ader 24 ist mit einem aushärtenden Kunststoff 30 ausgegossen.

Der Kunststoff 30 bildet einen Einführtrichter 32, der koaxial an das proximale Ende des Kanülenrohres 10 anschließt und sich von dem Innendurchmesser des Kanülenrohres 10 in proximaler Richtung auf den Durchmesser der Innenbohrung des Ansatzes 18 erweitert. An diesen Einführtrichter 32 in proximaler Richtung axial anschließend ist der Ansatz 18 als Luer-Lock-Anschluß 34 ausgebildet, der axial mit dem Kanülenrohr 10 fluchtet.

An dem freien Ende der Anschlußlitze 26 ist eine Stckverbinderbuchse 36 angebracht, mit welcher die Unipolar-Kanüle steckbar an einen elektrischen Nervenstimulator angeschlossen werden kann. Der Nervenstimulator sendet elektrische Stromimpulse von wenigen Milliampere aus, die über die Anschlußlitze 26, die Ader 24, die Anschlußbuchse 22 und das Kanülenrohr 10 zu dem blank liegenden distalen Endbereich 16 der Spitze 14 geleitet werden, um dort eine elektrische Nervenstimulation zur Lokalisierung der distalen Spitze 14 auszulösen.

An dem Außenumfang des Ansatzes 18 sind Griffbunde 38 angeformt. Eine Markierungskerbe 40 in einem der Griffbunde 38 macht die Winkelposition des Facettenschliffes 12 erkennbar.

Um die Unipolar-Kanüle zu plazieren, wird diese mittels der Steckverbinderbuchse 36 an den Nervenstimulator angeschlossen. Das Kanülenrohr 10 wird mittels der angeschliffenen Spitze 14 in die Nervenscheide eingestochen, wobei die jeweilige Position der Spitze 14 über die Elektrostimulation kontrolliert werden kann. Ist die distale Spitze 14 des Kanülenrohres 10 plaziert, so kann an den Luer-Lock-Anschluß 34 ein Zuspritzschlauch mittels eines Luer-Lock-Konnektors 42 angeschlossen werden, um ein Anästhetikum über das Kanülenrohr 10 einzuleiten. Alternativ kann an den Luer-Lock-Anschluß 34 auch eine Spritze konnektiert werden, um die Lage der distalen Spitze 14 durch Aspiration zu kontrollieren oder um über diese Spritze ein Anästhetikum einzuspritzen. Soll für eine Langzeitanästhesie ein Katheter gelegt werden, so wird dieser - gegebenenfalls nach Dekonnektieren des Zuspritzschlauches oder der Spritze - durch den Luer-Lock-Anschluß 34 axial eingeführt, wobei der Einführtrichter 32 die Katheterspitze in das Kanülenrohr 10 führt. Die Katheterspitze tritt durch das offene distale Ende des Kanülenrohres 10 axial aus und wird in die gewünschte Position gebracht. Ist der Katheter plaziert, so kann die Unipolar-Kanüle nach hinten von dem Katheter abgezogen werden, wobei der Katheter in seiner Position verbleibt.

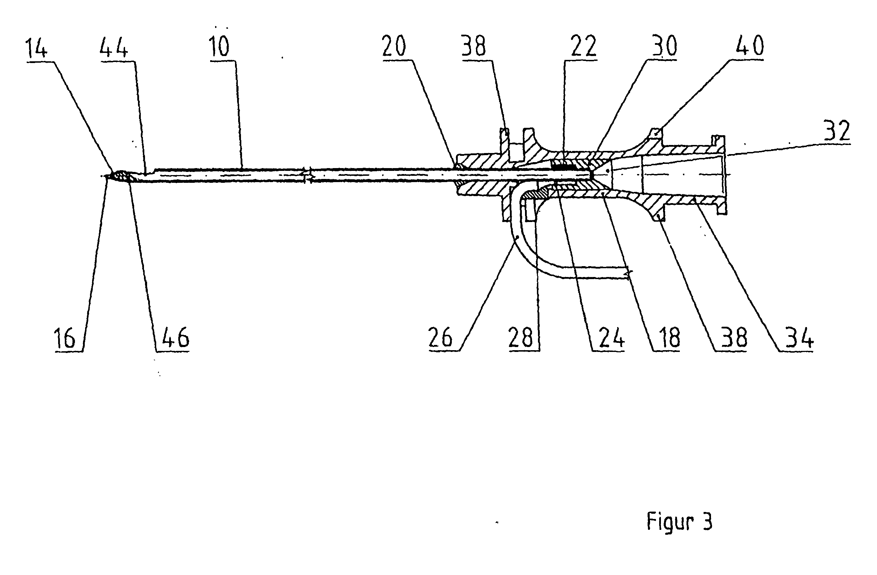

Figur 3 zeigt eine andere Ausführung der Unipolar-Kanüle. Soweit diese mit der vorstehend beschriebenen Ausführung übereinstimmt, sind dieselben Bezugszahlen verwendet und auf die vorstehende Beschreibung wird Bezug genommen.

Zum Unterschied von dem Ausführungsbeispiel der Figuren 1 und 2 ist bei der Unipolar-Kanüle der Figur 3 die distale Spitze 14 als Sprotte-Spitze ausgebildet, wie diese in der DE 36 43 235 C1 beschrieben ist. Die distale Spitze 14 ist als geschlossene gewölbte konische Spitze ausgebildet. Seitlich hinter der konischen Spitze ist eine Austrittsöffnung 44 vorgesehen. Die Spitze 14 ist mittels eines aushärtenden Kunststoffes 46 gefüllt, so daß sich im Inneren des Kanülenrohres 10 eine Rampe bildet, welche die in dem Kanülenrohr 10 distal nach vorn geschobene Katheterspitze aus der Axialrichtung ablenkt, so daß der Katheter unter einem Winkel etwa 30° zur Achse des Kanülenrohres 10 seitlich aus der Austrittsöffnung 44 austritt. Das Kanülenrohr 10 ist bis zu der konisch gewölbten Spitze 14 isolierend beschichtet, so daß auch hier nur ein distaler Endbereich 16 der Spitze 14 mit einer maximalen Länge von 1 mm blank liegt.

### Bezugszeichenliste

- 10: Kanülenrohr
- 12: Facettenschliff
- 14: distale Spitze
- 16: Endbereich
- 18: Ansatz
- 20: Klebstoff
- 22: Anschlußbuchse
- 24: Ader
- 26: Anschlußlitze
- 28: Klebstoff
- 30: Kunststoff
- 32: Einführtrichter
- 34: Luer-Lock-Anschluß
- 36: Steckverbinderbuchse
- 38: Griffbund
- 40: Markierungskerbe
- 42: Luer-Lock-Konnektor
- 44: Austrittsöffnung
- 46: Kunststofframpe

## Patentansprüche

1. Unipolar-Kanüle für die kontinuierliche Leitungsanästhesie, mit einem elektrisch leitenden Kanülenrohr (10), mit einer distalen Spitze (14) des Kanülenrohres (10), mit einer im Bereich der Spitze (14) angeordneten Austrittsöffnung (12, 44), mit einem am proximalen Ende des Kanülenrohres (10) angeordneten Ansatz (18) und mit einem Anschluss (22, 24, 26) für eine Elektrostimulation, welcher durch den Mantel des Ansatzes (18) führt und im Bereich des Ansatzes (18) das Kanülenrohr (10) an dessen Umfang elektrisch kontaktiert,
**dadurch gekennzeichnet, dass** das Kanülenrohr (10) eine elektrisch isolierende äußere Beschichtung aufweist, welche sich von dem Ansatz (18) bis zu der Spitze (14) erstreckt und diese Spitze (14) zumindest in ihrem distalen Endbereich (16) freilässt, dass der Ansatz (18) eine axial fluchtend in das Kanülenrohr (10) führende Einführöffnung mit einem sich koaxial gegen das proximale Ende des Kanülenrohres (10) verjüngenden Einführtrichter (32) zum Einführen eines Katheters aufweist, dass an dem Ansatz (18) ein Luer-Lock-Anschluss (34) zum axial fluchtenden Konnektieren einer Spritze oder eines Zuspritzschlauches ausgebildet ist und dass im Bereich der Spitze (14) des Kanülenrohres (10) eine Austrittsöffnung (12, 44) für den Katheter angeordnet ist.

2. Unlpolar-Kanüle nach Anspruch 1,
**dadurch gekennzeichnet, dass** der elektrisch kontaktierende Anschluß eine auf den Umfang des KanGlenrohres (10) aufgepreßte Anschlußbuchse (22) aufweist, mit welcher eine Ader (24) eine Anschlußlitze (26) verlötet ist.

3. Unipolar-Kanüle nach Anspruch 2,
**dadurch gekennzeichnet, dass** die Ader (24) achsparallel an dem Kanülenrohr (10) anliegt und die Anschlußlitze (26) radial durch den Ansatz (18) nach außen geführt ist.

4. Unipolar-Kanüle nach einem-deransprüche 1 bis 3, **dadurch gekennzeichet, dass** das Kanülenrohr (10) mit seinem proximalen Ende koaxial In den Ansatz (18) eingepaßt ist und dass ein Ringraum zwischen dem proximalen Ende des Kanülenrohres (10) mit dem elektrisch kontaktierenden Anschluß (22, 24) und einer Innenwandung des Ansatzes (1B) mit Kunststoff (30) ausgegossen ist.

5. Unipolar-Kanüle nach einem der vorhergehenden Ansprüche,
**dadurch gekennzeichnet, dass** das proximale Ende des Ansatzes (18) als Luer-Lock-Anschluß (34) ausgebildet ist.

6. Unipolar-Kanüle nach einem der vorhergehenden Anspruche,
**dadurch gekennzeichnet, dass** der blank liegende Endbereich (16) der distalen Spitze (14) des Kanülenrohres (10) eine axiale Länge von maximal 1 mm aufweist.

7. Unipolar-Kanüle nach einem der Ansprüche 1 bis 6, **dadurch gekennzeichnet, dass** die distale Spitze (149 des Kanülenrohres (10) durch einen Facettenschliff (12) gebildet Ist.

8. Unipolar-Kanüle nach Anspruch 7,
**dadurch gekennzeichnet, dass** der Facettenschliff (12) unter einem Winkel von etwa 45 ° gegen die Achse des Kanülenrohres (10) geneigt ist.

9. Unipolar-Kanüle nach einem der Ansprüche 1 bis 6, **dadurch gekennzeichnet, dass** die distale Spitze (14) des Kanülenrohres (10) als geschlossene konisch gewölbte Spitze mit einer seitlich hinter dieser Spitze angeordneten Austrittsöffnung (44) (sogenannte Sprotte-Spitze) ausgebildet ist.

10. Unipolar-Kanüle nach Anspruch 9,
**dadurch gekennzeichnet dass** im inneren des distalen Endes des Kanülenrohres (10) eine Rampe (46) ausgebildet ist, die zu der seitlichen Austrittsöffnung (44) führt.

## Claims

1. A unipolar cannula for continuous conduction anaesthesia, with an electrically conductive cannula tube (10), with a distal tip (14) of the cannula tube (10), with an exit opening (12, 44) arranged in the vicinity of the tip (14), with an attachment (18) arranged at the proximal end of the cannula tube (10) and with a connection (22, 24, 26) for electrical stimulation, which leads through the cover of the attachment (18) and in the vicinity of the attachment (18) electrically contacts the cannula tube (10) on the periphery thereof, **characterized in that** the cannula tube (10) has an electrically insulating external coating which extends from the attachment (18) as far as the tip (14) and leaves this tip (14) exposed at least in its distal end region (16), the attachment (18) has an insertion opening leading into the cannula tube (10) in an axially aligned manner and with an insertion funnel (32) tapering coaxially towards the proximal end of the cannula tube (10) for the insertion of a catheter, a luer lock connection (34) for connecting a syringe or an injection tube in an axially aligned manner is formed on the attachment (18), and an exit opening (12, 44) for the catheter is arranged in the vicinity of the tip (14) of the cannula tube (10).

2. A unipolar cannula according to claim 1, **characterized in that** the electrically contacting attachment (18) has an attachment bush (22) which is pressed onto the periphery of the cannula tube (10) and to which a wire (24) of an attachment cable (26) is soldered.

3. A unipolar cannula according to claim 2, **characterized in that** the wire (24) rests axially parallel against the cannula tube (10) and the attachment cable (26) is led radially through the attachment (18) to the outside.

4. A unipolar cannula according to any one of claims 1 to 3, **characterized in that** the cannula tube (10) is fitted with its proximal end coaxially in the attachment (18), and an annular space between the proximal end of the cannula tube (10) with the electrically contacting connection (22, 24) and an inner wall of the attachment (18) is lined with plastics material (30).

5. A unipolar cannula according to any one of the preceding claims, **characterized in that** the proximal end of the attachment (18) is designed in the form of a luer lock connection (34).

6. A unipolar cannula according to any one of the preceding claims, **characterized in that** the exposed end region (16) of the distal tip (14) of the cannula tube (10) has an axial length of a maximum of 1 mm.

7. A unipolar cannula according to any one of claims 1 to 6, **characterized in that** the distal tip (14) of the cannula tube (10) is formed by a facet cut (12).

8. A unipolar cannula according to claim 7, **characterized in that** the facet cut (12) is inclined at an angle of approximately 45° with respect to the axis of the cannula tube (10).

9. A unipolar cannula according to any one of claims 1 to 6, **characterized in that** the distal tip (14) of the cannula tube (10) is constructed in the form of a closed, conically convex tip with an exit opening (44) (a so-called Sprotte tip) arranged laterally behind this tip.

10. A unipolar cannula according to claim 9, **characterized in that** a ramp (46), which leads to the lateral exit opening (44), is formed in the interior of the distal end of the cannula tube (10).

## Revendications

1. Canule unipolaire pour l'anesthésie de conduction continue, avec un tube de canule (10) électroconducteur, avec une pointe (14) distale du tube de canule (10), avec une ouverture de sortie (12, 44) placée dans la zone de la pointe (14), avec un prolongement (18) placé à l'extrémité proximale du tube de canule (10) et avec un raccord (22, 24, 26) pour une stimulation électrique, lequel passe à travers l'enveloppe du prolongement (18) et met le tube de canule (10) sur son pourtour en contact électrique dans la zone du prolongement (18),
**caractérisée en ce que**
le tube de canule (10) présente un revêtement extérieur électriquement isolant qui s'étend du prolongement (18) jusqu'à la pointe (14) et laisse libre cette pointe (14) au moins dans sa zone terminale (16) distale, le prolongement (18) présente une ouverture d'insertion qui conduit de façon alignée axialement dans le tube de canule (10) et qui a un cône d'entrée (32) se rétrécissant coaxialement vers l'extrémité proximale du tube de canule (10) et servant à insérer un cathéter un raccord rapide "Luer Lock" (34) est formée sur le prolongement (18) pour permettre de connecter, de façon alignée axialement, une seringue ou un tube d'injection et une ouverture de sortie (12, 44) pour le cathéter est placée dans la zone de la pointe (14) du tube de canule (10).

2. Canule unipolaire selon la revendication 1,
**caractérisée en ce que**
le raccord électroconducteur présente une douille de raccordement (22) pressée sur le pourtour du tube de canule (10) avec laquelle est brasé un fil (24) d'un toron de raccordement (26).

3. Canule unipolaire selon la revendication 2,
**caractérisée en ce que**
le fil (24) s'appuie sur le tube de canule (10) parallèlement à l'axe et le toron de raccordement (26) est guidé radialement vers l'extérieur à travers le prolongement (18).

4. Canule unipolaire selon l'une des revendications 1 à 3,
**caractérisée en ce que**
le tube de canule (10) est ajusté avec son extrémité proximale coaxialement dans le prolongement (18), et un espace annulaire entre l'extrémité proximale du tube de canule (10) avec le raccordement (22, 24) assurant le contact électrique et une paroi intérieure du prolongement (18) est scellé avec une matière synthétique (30).

5. Canule unipolaire selon l'une des revendications précédentes,
**caractérisée en ce que**
l'extrémité proximale du prolongement (18) est formée comme raccord rapide "Luer-Lock" (34).

6. Canule unipolaire selon l'une des revendications précédentes,
**caractérisée en ce que**
la zone terminale (16), dénudée, de la pointe (14) distale du tube de canule (10) présente une longueur axiale de 1 mm au maximum.

7. Canule unipolaire selon l'une des revendications 1 à 6,
**caractérisée en ce que**
la pointe (14) distale du tube de canule (10) est formée par une surface polie en biseau (12).

8. Canule unipolaire selon la revendication 7,
**caractérisée en ce que**
la surface polie en biseau (12) est inclinée selon un angle d'environ 45° par rapport à l'axe du tube de canule (10).

9. Canule unipolaire selon l'une des revendications 1 à 6,
**caractérisée en ce que**
la pointe (14) distale du tube de canule (10) est formée en tant que pointe fermée et recourbée coniquement avec une ouverture de sortie (44) placée latéralement derrière cette pointe.

10. Canule unipolaire selon la revendication 9,
**caractérisée en ce qu'**
une rampe (46) qui aboutit à l'ouverture de sortie (44) est formée à l'intérieur de l'extrémité distale du tube de canule (10).
